Europäisches Patentamt

European Patent Office

Office européen des brevets

(11) Veröffentlichungsnummer: **0 379 629**
**A1**

## (12) EUROPÄISCHE PATENTANMELDUNG

(21) Anmeldenummer: **89104202.0**

(22) Anmeldetag: **09.03.89**

(51) Int. Cl.⁵: **A61B 6/00**

(30) Priorität: **27.01.89 DE 8900948 U**

(43) Veröffentlichungstag der Anmeldung:
**01.08.90 Patentblatt 90/31**

(84) Benannte Vertragsstaaten:
**BE DE FR GB IT**

(71) Anmelder: **Siemens Aktiengesellschaft**
**Wittelsbacherplatz 2**
**D-8000 München 2(DE)**

(72) Erfinder: **Heuck, Friedrich, Prof. Dr.**
**Hermann-Kurz-Strasse 5**
**D-7000 Stuttgart 1(DE)**
Erfinder: **Heinz, Lothar, Dipl.-Ing. (FH)**
**Weingasse 63**
**D-8524 Neunkirchen(DE)**

(54) **Röntgendiagnostikgerät für Mammographieaufnahmen.**

(57) Ein Röntgendiagnostikgerät für Mammographie-aufnahmen soll so ausgeführt werden, daß mit die-sem sowohl Mammographieaufnahmen als auch Auf-nahmen einer Hand einer Untersuchungsperson bei jeweils optimalen Aufnahmeergebnissen angefertigt werden können. Erfindungsgemäß ist eine Aufnah-mevorrichtung mit Kassettenfach (10, 11, 17, 18, 23) derart ausgebildet, daß in einer ersten Aufnahmeposition das vordere Ende (12, 16, 24) des Kassettenfaches (10, 17, 23) und in einer zweiten Aufnahmeposition der mittlere Bereich des Kassettenfaches (11, 18, 23) vertikal unter dem Fokus (F) liegt.

FIG 2

EP 0 379 629 A1

## Röntgendiagnostikgerät für Mammographieaufnahmen

Die Erfindung betrifft ein Röntgendiagnostikgerät für Mammographieaufnahmen mit einem Röntgenstrahler mit einem Fokus zum Aussenden von Röntgenstrahlung und mit einer verstellbaren, auf den Röntgenstrahler ausrichtbaren Aufnahmevorrichtung mit Kassettenfach.

Ein Röntgendiagnostikgerät der obengenannten Art ist beispielsweise aus der DE-OS 33 19 622 bekannt. Bei diesem Röntgendiagnostikgerät sind zwei Aufnahmetische wahlweise auf den Röntgenstrahler ausrichtbar. In der Aufnahmeposition der Aufnahmetische liegt jeweils das vordere Ende des Kassettenfaches vertikal unter dem Fokus. Diese Ausrichtung ist für Mammographieaufnahmen wichtig, damit die Röntgenstrahlung die Brustwand tangiert.

Die in der Mammographie verwendete niederenergetische Röntgenstrahlung wird auch für die sogenannte Weichstrahldiagnostik (z.B. Weichstrahl-Immersions-Technik der Handaufnahme) benötigt. Es lassen sich somit durch Röntgenaufnahmen der Hände Rheuma- und Osteoporoserkrankungen früh erkennen. Das Anfertigen einer Handaufnahme mit einem Gerät der eingangs genannten Art bringt nur unbefriedigende Aufnahmeergebnisse, da insbes. der Fingerbereich der Hand durch die dort schräg auftreffende Röntgenstrahlung nicht ausreichend gut darstellbar ist. Es treten Verzeichnungs- und Projektionsfehler auf.

Aufgabe der Erfindung ist es daher, ein Röntgendiagnostikgerät der eingangs genannten Art so auszuführen, daß mit diesem Mammographieaufnahmen und Handaufnahmen angefertigt werden kön nen, bei denen jeweils optimale Aufnahmeergebnisse erhalten werden.

Die Aufgabe wird erfindungsgemäß dadurch gelöst, daß die Aufnahmevorrichtung derart ausgebildet ist, daß in einer ersten Aufnahmeposition das vordere Ende des Kassettenfaches und in einer zweiten Aufnahmeposition der mittlere Bereich des Kassettenfaches vertikal unter dem Fokus liegt.

In der ersten Aufnahmeposition können somit optimale Ergebnisse für eine Mammographieaufnahme erhalten werden, wo hingegen in der zweiten Aufnahmeposition optimale Ergebnisse für eine Handaufnahme erhalten werden.

Eine zweckmäßige Ausgestaltung der Erfindung besteht darin, daß die Aufnahmevorrichtung zwei Aufnahmetische aufweist, die zusammen mit einem um eine horizontale Achse schwenkbaren Träger eine U-förmige Aufnahmeeinheit bilden, die den Röntgenstrahler und eine Kompressionsvorrichtung außen umgreift, in der sich die Aufnahmetische gegenüberliegen, und wobei die beiden Schenkel der Aufnahmeeinheit, die die Aufnahmetische bilden, unterschiedlich lang sind. Bei dieser Ausgestaltung genügt ein Schwenken der Aufnahmetische um die horizontale Achse, so daß die beiden Aufnahmetische in ihre jeweilige Aufnahmeposition verstellt werden können.

Ausführungsbeispiele der Erfindung sind nachfolgend anhand der Figuren näher erläutert. Dabei zeigt:

Figur 1 ein erstes Ausführungsbeispiel eines Röntgendiagnostikgerätes nach der Erfindung,

Figur 2 ein zweites Ausführungsbeispiel eines Röntgendiagnostikgerätes nach der Erfindung und

Figur 3 ein weiteres Ausführungsbeispiel eines Röntgendiagnostikgerätes nach der Erfindung.

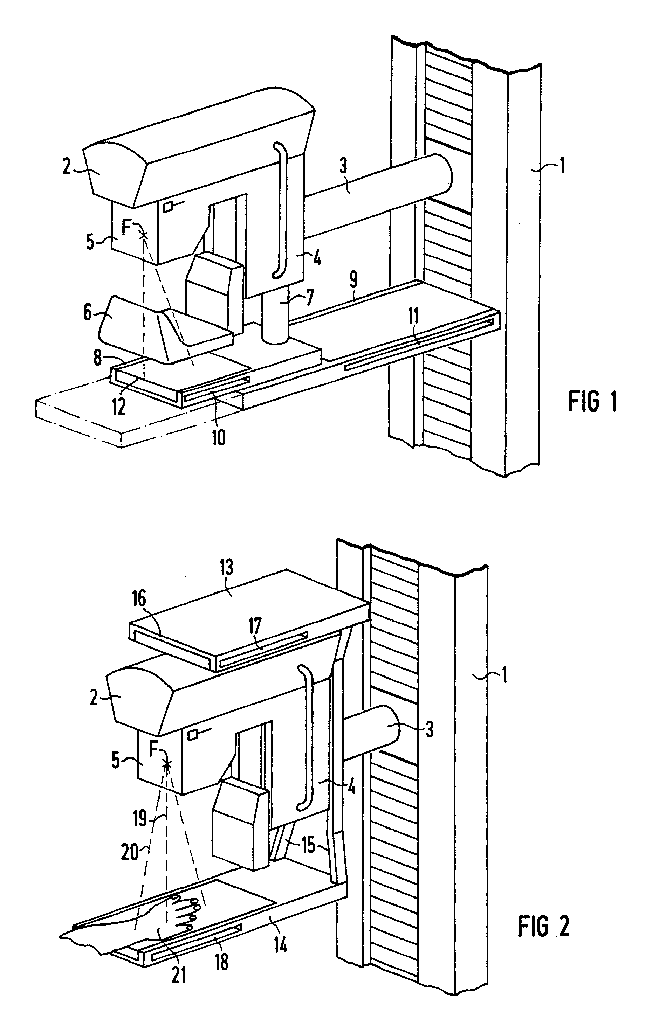

In der Figure 1 ist ein Stativ 1 dargestellt, mit dem ein Röntgenstrahler 2 mit Hilfe einer horizontal ausgerichteten Achse 3 höhenverstellbar ist. Die Achse 3 trägt den Röntgenstrahler 2 mit Hilfe eines Blockes 4, an dem eine Primärstrahlenblende 5 angeordnet ist. Der Block 4 trägt ferner eine motorisch in vertikaler Richtung verstellbare Kompressionsplatte 6.

Am Block 4 ist eine vertikale Achse 7 befestigt, um die zwei Aufnahmetische 8 und 9 mit jeweils einem Kassettenfach 10, 11 für Röntgenfilme schwenkbar gelagert sind. In der Figur 1 befindet sich der Aufnahmetisch 8, der für Mammographieaufnahmen benötigt wird, in der Aufnahmeposition. In dieser Position liegt das vordere Ende 12 des Kassettenfaches 10 vertikal unter dem Fokus F. Für eine Mammographieaufnahme wird das Röntgenstrahlenbündel des Röntgenstrahlers 2 durch die Primärstrahlenblende 5 auf den Bereich des Kassettenfaches 10 eingeblendet. Mit dieser Anordnung kann bei möglichst geringer Strahlenbelastung der Untersuchungsperson eine möglichst verzeichnungs- und abbildungsfehlerfreie Mammographieaufnahme angefertigt werden. Die Figur 1 zeigt, daß der Aufnahmetisch 9 in einer Parkposition liegt. Soll mit dem Aufnahmetisch 9 gearbeitet werden, so werden die Aufnahmetische 8 und 9 um die Achse 7 so verschwenkt, daß der Aufnahmetisch 9 die Aufnahmeposition und der Aufnahmetisch 8 die Parkposition einnimmt. Wenn sich der Aufnahmetisch 9 in der Aufnahmeposition befindet, liegt der mittlere Bereich des Kassettenfaches 11 vertikal unter dem Fokus F, was beispielsweise dadurch bewirkt wird, daß der Aufnahmetisch 9 länger ist als der Aufnahmetisch 8. Das Röntgenstrahlenbündel wird durch die Primärstrahlenblende 5 entsprechend der geänderten Lage des Kassettenfaches 11 eingeblendet. In dieser Aufnahmeposition können beispielsweise Handaufnahmen angefertigt werden, die weitestgehend projektions-und verzeichnungsfehlerfrei sind.

Die Figur 2 zeigt ein weiteres Ausführungsbeispiel eines Röntgendiagnostikgerätes nach der Erfindung. Teile, die bereits in der Figur 1 mit Bezugzeichen versehen wurden, erhalten in der Figur 2 die gleichen Bezugszeichen. Zwei Aufnahmetische 13 und 14 liegen entgegen dem Ausführungsbeispiel nach der Figur 1 in der Figur 2 einander gegenüber. Sie bilden mit einem um eine horizontale Achse 3 schwenkbaren Träger 15 eine U-förmige Aufnahmeeinheit, deren Schenkel, die von den Aufnahmetischen 13 und 14 gebildet werden, unterschiedlich sind. Die Aufnahmeeinheit 13, 14, 15 umgreift den Röntgenstrahler 2 außen. Die Figur 2 zeigt den Aufnahmetisch 14 in der Aufnahmeposition und den Aufnahmetisch 13 in einer Parkposition oberhalb des Röntgenstrahlers 2. Soll der Aufnahmetisch 13 in die Aufnahmeposition verstellt werden, so wird der Träger 15 auf der Achse 3 um 180° geschwenkt, so daß er die Position des Aufnahmetisches 14 und der Aufnahmetisch 14 die Position des Aufnahmetisches 13 einnimmt. In der Aufnahmeposition des Aufnahmetisches 13 liegt dann das vordere Ende 16 des Kassettenfaches 17 vertikal unter dem Fokus F. Die Figur 2 zeigt die Aufnahmeposition des Aufnahmetisches 14, in der mit dem Röntgendiagnostikgerät Handaufnahmen einer Untersuchungsperson angefertigt werden können. Hier befindet sich, wie die Figur 2 zeigt, der Zentralstrahl 19 des Röntgenstrahlenbündels 20 des Röntgenstrahlers 2 im mittleren Bereich des Kassettenfaches 18 des Aufnahmetisches 14. Es werden somit gute Ergebnisse für Handaufnahmen erhalten.

Zur Anfertigung einer Röntgenaufnahme der Brust wird gemäß der obigen Figurenbeschreibung der Aufnahmetisch 8 bzw. 13 in die Aufnahmeposition verstellt. Im Kassettenfach 10 des Aufnahmetisches 8 bzw. im Kassettenfach 17 des Aufnahmetisches 13 befindet sich eine Filmkassette mit einem Röntgenfilm zur Belichtung. Nachdem die Brust auf dem Aufnahmetisch 8 bzw. 13 gelagert wurde, erfolgt die Kompression der Brust durch Verstellen der Kompressionsplatte 6 nach unten. Eine Kompressionsplatte 6 ist selbstverständlich auch für ein Röntgendiagnostikgerät gemäß der Figur 2 vorgesehen. Nach anschließend erfolgter Einschaltung des Röntgenstrahlers 2 kann die Filmkassette mit dem belichteten Röntgenfilm aus dem Kassettenfach 10 bzw. 17 entnommen werden.

Zur Früherkennung von Rheuma- oder Osteoporoseerkrankungen können Handaufnahmen angefertigt werden. Hierzu kann, gemäß der obigen Figurenbeschreibung, der Aufnahmetisch 9 bzw. 14 in die Aufnahmeposition und der Aufnahmetisch 8 bzw. 13 in die Parkposition verstellt werden. Die Figur 2 zeigt, daß sich der Aufnahmetisch 14 in der Aufnahmeposition befindet. Nachdem die Kompressionsplatte 6 aus dem Strahlengang entfernt wurde, wird die Hand 21 einer nicht gezeigten Untersuchungsperson auf den Aufnahmetisch 14 bzw. 9 aufgelegt und so positioniert, daß der mittlere Handbereich im mittleren Bereich des Kassettenfaches 18 bzw. 11 zu liegen kommt. Dieser Bereich liegt dann dem Fokus F in vertikaler Richtung gegenüber. Zur Anfertigung einer Handaufnahme wird der Röntgenstrahler 2, wie bereits oben erwähnt, zur Belichtung eines Röntgenfilmes in einer Filmkassette im Kassettenfach 18 bzw. 11 eingeschaltet.

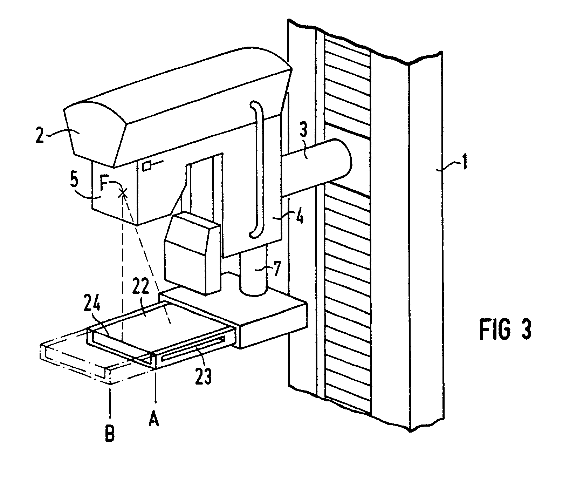

Die Erfindung schließt auch einen in der Figur 3 gezeigten Aufnahmetisch 22 mit einem Kassettenfach 23 ein, der in Richtung seiner Längsachse verstellbar ist. Elemente, die bereits in den Figuren 1 und 2 mit Bezugszeichen versehen wurden, erhalten in der Figur 3 die gleichen Bezugszeichen. In einer ersten Position A liegt zur Anfertigung einer Mammographieaufnahme das vordere Ende 24 des Kassettenfaches 23 vertikal unter dem Fokus F des Röntgenstrahlers 2. Nach Verstellung des Aufnahmetisches 22 in Richtung seiner Längsachse in eine Position B können Handaufnahmen angefertigt werden, wobei dann der mittlere Bereich des Kassettenfaches 23 vertikal unter dem Fokus F des Röntgenstrahlers 2 liegt.

Bei allen Ausführungsformen der Erfindung kann die Einblendung des Röntgenstrahlenbündels durch die Primärstrahlenblende 5 automatisch in Abhängigkeit vom gewählten Aufnahmetisch 8, 9, 13, 14 bzw. von der gewählten Aufnahmeposition (A, B) des Aufnahmetisches 22 bzw. in Abhängigkeit von der Anordung des Kassettenfaches 10, 11, 17, 18, 23 relativ zum Fokus F erfolgen. Zudem kann im Bereich der Aufnahmetische 8, 9, 13, 14, 22 jeweils ein nicht gezeigtes Streustrahlenraster vorgesehen sein.

## Ansprüche

1. Röntgendiagnostikgerät für Mammographieaufnahmen mit einem Röntgenstrahler (2) mit einem Fokus (F) zum Aussenden von Röntgenstrahlung und mit einer verstellbaren, auf den Röntgenstrahler (2) ausrichtbaren Aufnahmevorrichtung mit Kassettenfach (10, 11, 17, 18, 23), **dadurch gekennzeichnet**, daß die Aufnahmevorrichtung derart ausgebildet ist, daß in einer ersten Aufnahmeposition das vordere Ende (12, 16, 24) des Kassettenfaches (10, 17, 23) und in einer zweiten Aufnahmeposition der mittlere Bereich des Kassettenfaches (11, 18, 23) vertikal unter dem Fokus (F) liegt.

2. Röntgendiagnostikgerät nach Anspruch 1, **dadurch gekennzeichnet**, daß die Aufnahmevorrichtung von einem in Richtung seiner Längsachse verstellbaren Aufnahmetisch (22) gebildet ist.

3. Röntgendiagnostikgerät nach Anspruch 1, wobei die Aufnahmevorrichtung zwei Aufnahmetische (13, 14) aufweist, die zusammen mit einem um eine horizontale Achse (3) schwenkbaren Träger (15) eine U-förmige Aufnahmeeinheit bilden, wobei ein Schenkel der Aufnahmeeinheit den Röntgenstrahler (2) außen umgreift, und wobei sich die Aufnahmetische (13, 14) gegenüberliegen, **dadurch gekennzeichnet**, daß die beiden Schenkel der Aufnahmeeinheit, die die Aufnahmetische (13, 14) bilden, unterschiedlich lang sind.

4. Röntgendiagnostikgerät nach Anspruch 1, wobei die Aufnahmevorrichtung zwei Aufnahmetische (8, 9) aufweist, die um eine vertikale Achse (7) schwenkbar sind, **dadurch gekennzeichnet**, daß die Aufnahmetische (8, 9) unterschiedlich lang sind.

5. Röntgendiagnostikgerät nach einem der Ansprüche 1 bis 4, **dadurch gekennzeichnet**, daß die Einblendung des Röntgenstrahlenbündels automatisch in Abhängigkeit von der Anordnung des Kassettenfaches (10, 11, 17, 18, 23) relativ zum Fokus (F) erfolgt.

FIG 1

FIG 2

FIG 3

| | Europäisches Patentamt | EUROPÄISCHER RECHERCHENBERICHT | Nummer der Anmeldung |
|---|---|---|---|
| | | | EP 89 10 4202 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 127 073 (SIEMENS AG) <br> * Seite 3, Zeile 8 - Seite 4, Zeile 29; Figuren 1,2 * | 1 | A 61 B 6/00 |
| A | | 3,4 | |
| Y | WO-A-8 703 795 (MEDICAL PRODUCTS AB) <br> * Seite 7, Zeilen 1-9; Seite 19, Zeile 17 - Seite 21, Zeile 21; Figuren 1,13-15 * | 1 | |
| A | | 5 | |

|  |
|---|
| RECHERCHIERTE SACHGEBIETE (Int. Cl.4) |
| A 61 B 6/00 <br> H 05 G 1/00 <br> G 03 B 42/00 |

**Der vorliegende Recherchenbericht wurde für alle Patentansprüche erstellt**

| Recherchenort | Abschlußdatum der Recherche | Prüfer |
|---|---|---|
| BERLIN | 19-02-1990 | WEIHS J.A. |